# FASCICULE DE BREVET EUROPEEN

(11) **EP 2 456 550 B1**
(45) Date de publication et mention de la délivrance du brevet: **05.04.2017**
(21) Numéro de dépôt: 10750148.8
(22) Date de dépôt: 20.07.2010
(51) Int. Cl.: B01J 13/04

(54) **PROCEDE DE FABRICATION DE CAPSULES**
VERFAHREN ZUR HERSTELLUNG VON KAPSELN
METHOD FOR MANUFACTURING CAPSULES

(30) Priorité: 20.07.2009 FR 0955035
(43) Date de publication de la demande: 30.05.2012
(73) Titulaire: Centre National de la Recherche Scientifique (C.N.R.S.), 75016 Paris (FR); Université de Montpellier, 34090 Montpellier (FR)
(72) Inventeur: MASSIERA, Gladys, F-34090 Montpellier (FR); LOISEAU, Etienne, F-12100 Millau (FR); ABKARIAN, Manouk, F-34000 Montpellier (FR)
(74) Mandataire: Cabinet Plasseraud
(86) Numéro de dépôt international: PCT/FR2010/000523
(87) Numéro de publication internationale: WO 2011/010017

(56) Documents cités:
- SOPHIE PAUTOT, E.A.: "Production of Unilamellar Vesicles Using an Inverted Emulsion" LANGMUIR, vol. 19, no. 7, 19 février 2003 (2003-02-19), pages 2870-2879, XP002575093 cité dans la demande
- UMBANHOWAR P B ET AL: "Monodisperse Emulsion Generation via Drop Break Off in a Coflowing Stream" LANGMUIR, AMERICAN CHEMICAL SOCIETY, WASHINGTON, DC, US, vol. 16, 14 octobre 1999 (1999-10-14), pages 347-351, XP002552872 ISSN: 0743-7463

## Description

La présente invention concerne le domaine de la fabrication de capsules utiles notamment pour encapsuler un échantillon d'intérêt ou comme outil de calibrage de matériel d'analyse.

Plusieurs méthodes pour produire des capsules ont déjà été décrites dans la littérature. Par exemple, pour un type de capsules dont l'enveloppe est une bicouche, il existe des méthodes d'hydratation, en particulier l'électroformation, qui consistent à hydrater un film de lipides et qui permettent de produire des capsules de bonne qualité, mais avec un rendement très faible et une grande polydispersité dans la taille des capsules obtenues. Il existe aussi des méthodes de production de double émulsion suivie d'une évaporation du solvant compris entre les deux monocouches amphiphiles ; ou encore des méthodes de production d'une émulsion calibrée par microfluidique puis de dispersion de l'émulsion produite dans une solution aqueuse d'eau et d'éthanol.

Toutefois, aucune des méthodes qui précèdent ne permettent de fabriquer, dans des délais courts, des quantités importantes de capsules de taille contrôlée, de bonne qualité, c'est à dire sphériques et sans défaut, avec une bonne reproductibilité de la méthode. Par ailleurs, un grand nombre des méthodes de l'art intérieur font intervenir des solvants organiques, souvent toxiques, qu'il faut éliminer avant de pouvoir utiliser les capsules.

La Demanderesse s'est particulièrement intéressée à une méthode décrite dans Pautot et al, Langmuir 2003, 19, 2870-2879, dans laquelle une émulsion inversée est préparée par mixage d'une phase aqueuse et d'une première phase huileuse en présence de surfactant : les molécules de surfactant présentes dans l'huile s'adsorbent à la surface de la goutte aqueuse et forment une monocouche. Une double phase constituée d'une phase aqueuse, d'une interface et d'une phase intermédiaire est ensuite préparée comme suit : on verse une seconde phase huileuse également additionnée de surfactant sur une phase aqueuse, et on laisse une interface se former. L'huile étant moins dense, elle reste au dessus de l'eau. L'émulsion préparée initialement est versée sur cette double phase, et les vésicules, plus lourde que la phase huileuse, sédimentent vers la phase aqueuse. En traversant l'interface, elles se couvrent d'une seconde couche lipidique. Dans PNAS 2003, 19, vol. 100, 10718-10721, Pautot et al. décrit la fabrication de vésicules à membrane asymétriques à partir du même type d'émulsion que celle citée ci-dessus, et du même type de phase intermédiaire, puis l'application d'une force centrifuge à 120 x g pendant 10 minutes pour transférer les gouttelettes d'eau à travers l'interface huile/eau vers la phase aqueuse inférieure.

Toutefois, la méthode de Pautot et al., avec ou sans centrifugation, ne permet pas d'obtenir des suspensions de vésicules ayant une polydispersité satisfaisante. Par ailleurs, les méthodes de Pautot et al., ne sont pas suffisamment reproductibles pour être mises en oeuvre industriellement.

Sophie PAUTOT, E.A.: "Production of Unilamellar Vesicles Using an lnverted Emulsion", LANGMUIR, vol. 19, no. 7, 19 février 2003 (2003-02-19), pages 2870-2879, XP002575093, décrit un procédé de fabrication de vésicules, comprenant la mise en contact d'une émulsion inverse avec une phase d'huile se trouvant dans un tube. En revanche, ce document ne décrit pas un procédé de fabrication en continu et en une seule étape.

Dans le domaine de production de capsules, il est connu que la productivité de la méthode est un facteur industriel important. Des procédés industriellement efficaces et reproductibles, permettant de pouvoir contrôler le coeur de la capsule et la formation des capsules elles-mêmes, dans leur nature, dans leur qualité, dans leur quantité et dans leur indice de polydispersité de manière à contrôler la production, sont donc toujours recherchés.

L'invention a donc pour objet un nouveau procédé de fabrication de capsules, qui est un procédé en une seule étape, économique, robuste et qui permet un contrôle fin de la taille des capsules produites et un haut rendement. Le procédé selon l'invention permet d'obtenir des capsules qui sont sans défaut. La mise en oeuvre du procédé selon l'invention évite toute coalescence des capsules entre elles.

Plus précisément, l'invention concerne un procédé de fabrication en une seule étape de capsules de 1 à 100 microns, ayant un indice de polydispersité inférieur à 10%, tel que défini dans la revendication 1, ledit procédé comprenant la mise en contact de gouttelettes de tailles homogènes d'une composition aqueuse, émises en continu, avec une phase intermédiaire se trouvant dans une chambre en rotation, ladite chambre comprenant une phase aqueuse et une phase intermédiaire, ces deux phases formant une interface, à travers laquelle lesdites gouttelettes sont forcées sous l'effet de la force centrifuge générée par la rotation de la chambre, puis la récupération d'une suspension aqueuse de capsules. Sous l'effet de la force centrifuge, l'interface est verticale. Avantageusement, la phase intermédiaire est une dispersion de molécules amphiphiles dans un solvant non miscible à l'eau et de densité inférieure à celle de l'eau.

Suivant un mode de réalisation préféré de l'invention, la phase aqueuse a une densité inférieure d'au moins 0.5 à 25g/l à la densité de la composition aqueuse ; avantageusement, la phase aqueuse et la composition aqueuse sont iso-osmotiques. De manière particulière, la composition aqueuse est injectée au moyen de capillaires à un débit de 100 à 500 µl/h, de préférence 150 à 250 ul/h ou à une pression fixe de 80 à 500 mbar, de préférence 100 à 200 mbar (selon la taille du capillaire) dans la phase intermédiaire se trouvant dans la chambre en rotation, à une distance fixe et déterminée de l'interface, ladite injection résultant, par la rotation, en la production de gouttelettes à intervalles réguliers dans la phase intermédiaire par un mécanisme de goutte à goutte ou bien de jet selon la taille des gouttes que l'on souhaite produire. La polydispersité des gouttelettes produites est, selon le procédé de l'invention, dépendante de la taille du capillaire et du nombre capillaire de l'écoulement à l'extrémité du capillaire, ce nombre étant inférieur à 1, de préférence de 10⁻⁴ à 10⁻¹, très préférentiellement d'environ 0,5.10⁻³.

Dans un autre mode de réalisation de l'invention, la phase aqueuse sera injectée à partir d'un cylindre percé permettant de multiplier le nombre de points d'injections et donc le rendement.

Il est décrit un dispositif de mise en oeuvre du procédé selon l'invention, ce dispositif comprenant
▪ une chambre dans laquelle seront formées les capsules ; cette chambre étant susceptible d'être mise en rotation, ladite chambre comprenant une phase aqueuse et une phase intermédiaire, et
▪ des moyens de mettre en contact la composition aqueuse avec le contenu de la chambre.

Il est également décrit une suspension de capsules, susceptible d'être obtenue par le procédé de l'invention. Dans un mode de réalisation particulier les capsules peuvent être sédimentées sur un substrat pour former un réseau s'apparentant à un tissu artificiel. De préférence, ce tissu artificiel est un tapis de vésicules collées les unes aux autres. Ces capsules peuvent contenir des filaments d'actine. Ce tissu est obtenu à partir du procédé, par sédimentation de la suspension directement obtenue par le procédé selon l'invention.

Avantageusement, les capsules de la suspension de capsules comprennent une enveloppe et un coeur de composition aqueuse. Dans un premier mode de réalisation, l'épaisseur de l'enveloppe est comprise entre 1nm et 10 microns, préférentiellement entre deux fois la taille de la molécule amphiphile dispersée à 10 microns. Dans un premier mode de réalisation où l'enveloppe est une bicouche lipidique, l'épaisseur de l'enveloppe est comprise entre 1 à 100 nm, très préférentiellement de 5 à 20 nm ; dans un second mode de réalisation où l'enveloppe est constituée de phase intermédiaire, l'épaisseur de l'enveloppe est inférieure ou égale au rayon du coeur, et de préférence est comprise entre 100 nm à 10 microns. Dans ce second mode de réalisation, le volume de l'enveloppe peut avoir 3 à 10 fois, de préférence environ 7 fois, le volume du coeur.

Il est également décrit l'utilisation du procédé selon l'invention, pour encapsuler des compositions aqueuses comprenant ou constituées par des principes actifs pharmaceutiques, des actifs cosmétiques, des substances biologiques, par exemple des acides nucléiques, des protéines, des solutions colloïdales, des échantillons biologiques humains ou environnementaux, ou encore pour encapsuler des produits sanguins. Il est également décrit une suspension de capsules susceptible d'être obtenue par le procédé de l'invention, lesdites capsules encapsulant des principes actifs pharmaceutiques, des actifs cosmétiques, des acides nucléiques, des protéines, des échantillons biologiques humains ou environnementaux, ou encore des produits sanguins (suspension d'hémoglobine ou de substitut sanguin, ou tout produit sanguin labile ou stable, notamment du type concentré de globule rouge, concentré plaquettaire, plasma ; ou encore un médicament dérivé du sang du type notamment protéines coagulantes, immunoglobulines, albumine).

Il est également décrit l'utilisation du procédé selon l'invention pour la production de produits sanguins artificiels, notamment de substituts sanguins. Dans un premier mode de réalisation, l'enveloppe est de préférence une bicouche perméable aux gaz et imperméable à l'hémoglobine. Il est également décrit une suspension de capsules susceptible d'être obtenue par le procédé de l'invention, lesdites capsules encapsulant de l'hémoglobine et l'enveloppe de ladite capsule étant perméable aux gaz et imperméable à l'hémoglobine.

Il est également décrit l'utilisation d'une suspension de capsules comme outil de calibrage. En effet, le procédé selon l'invention permet un contrôle parfait de la taille et du contenu des capsules, permettant ainsi d'utiliser des suspensions de capsules selon l'invention comme outil de calibrage.

Au sens de la présente invention, le terme :
- « capsule » se réfère à une sphère limitée par une enveloppe susceptible de contenir une composition aqueuse, cette sphère ayant un diamètre de 1 à 100 microns, de préférence 5 à 80 microns, plus préférentiellement de 10 à 30 microns ; dans un mode de réalisation particulier, la capsule selon l'invention est une vésicule, à savoir une capsule dont l'enveloppe est une bicouche d'amphiphile, préférablement une bicouche lipidique ;
- « polydispersité » se réfère à la distribution de taille d'une population de capsules. Celle ci est obtenue par analyse d'images, en détectant les contours des capsules et en les ajustant avec un cercle. L'histogramme des diamètres des cercles permet d'obtenir la moyenne et la variance de la distribution, le rapport variance sur moyenne définissant l'indice de polydispersité. Plus ce rapport est petit, plus la distribution est étroite et plus la solution de capsules se rapproche d'une solution monodisperse de capsules.

- « Phase intermédiaire » se réfère à une composition liquide, susceptible d'être composée de plusieurs fluides de masses volumiques différentes, ladite phase intermédiaire étant :
- de masse(s) volumique(s) inférieure(s) à celle de la composition aqueuse et à celle de la phase aqueuse,
- non-miscible avec la composition aqueuse et/ou la phase aqueuse
- comprend ou est constituée par des molécules amphiphiles, qui peuvent être choisies parmi notamment des lipides, des polymères di-, tri- ou multi-blocs, des tensioactifs, des protéines.

Ainsi, l'invention a pour objet un procédé de fabrication de capsules ou d'une suspension de capsules de 1 à 100 microns, ayant un indice de polydispersité inférieur à 10%, de préférence inférieur à 9%, préférentiellement inférieur à 7 %, très préférentiellement d'environ 6%, en une seule étape. le procédé s'effectue en continu, la composition aqueuse peut-être injectée à partir du capillaire dans la phase intermédiaire avec une pression de 80 mbar à 500 mbar, préférentiellement de 100 à 200 mbar, et les gouttelettes de composition aqueuse sont arrachées de l'extrémité du capillaire par la force liée à la rotation du liquide (la phase intermédiaire) avec lequel l'extrémité du capillaire est en contact, situé dans la chambre. Ainsi, les gouttelettes arrivent séquentiellement dans la phase intermédiaire.

Cette phase intermédiaire est telle que le régime de l'écoulement du fluide autour du capillaire est à faible nombre capillaire (inférieur à 1), ce qui assure un régime de goutte-à-goutte de la composition aqueuse, ce régime étant dominé par la tension interfaciale entre la composition aqueuse et la phase intermédiaire, et donc une grande reproductibilité de la taille des gouttes, c'est-à-dire un faible indice de polydispersité des gouttes de composition aqueuse.

Chaque goutte arrachée du capillaire est soumise à la force centrifuge et s'éloigne rapidement du capillaire vers l'interface : ainsi, les phénomènes de coalescence sont évités, à tout le moins minimisés.

Suivant un premier mode de réalisation, la phase intermédiaire comprend ou consiste en deux couches de fluides, de densités différentes: une première couche moins dense et moins visqueuse que la seconde, permet de maintenir un nombre capillaire bas pour que le mécanisme de goutte à goutte de composition aqueuse soit dominé par la tension interfaciale ce qui permet d'obtenir une distribution de taille optimisée ; une seconde couche, qui est une dispersion de molécules amphiphiles.

Suivant un second mode de réalisation, on utilisera une phase intermédiaire comportant une couche unique, celle de la dispersion de molécules amphiphiles, de préférence de lipides.

L'épaisseur de la phase intermédiaire et la concentration en molécules amphiphiles de cette phase intermédiaire à couche unique, ou de la seconde couche de la phase intermédiaire bi-couche, sont deux paramètres dépendants. Ainsi, la concentration en molécules amphiphiles fixe le temps d'adsorption des molécules amphiphiles sur les gouttes de composition aqueuse jusqu'à saturation. L'épaisseur de cette couche de phase intermédiaire devra donc être choisie de façon à ce que les gouttes formées à partir du capillaire en vol dans la phase intermédiaire aient eu le temps de se couvrir jusqu'à saturation de molécules amphiphiles avant d'atteindre l'interface. Suivant un mode de réalisation préféré de l'invention, la concentration en molécules amphiphiles est de 0.05 à 5 mM, de préférence 0.1 à 1 mM, très préférentiellement environ 0,5 mM.

La composition aqueuse peut être toute composition d'intérêt, notamment du type contenant des substances biologiques, par exemple des acides nucléiques, des protéines, des échantillons biologiques humains (globules rouges, globules blancs, plaquettes...) ou environnementaux, des principes actifs pharmaceutiques, des actifs cosmétiques, des solutions colloïdales, etc. Suivant un mode de réalisation particulier de l'invention, la composition aqueuse est un produit sanguin, de préférence une suspension d'hémoglobine ou de substitut sanguin, ou tout produit sanguin labile ou stable, notamment du type concentré de globule rouge, concentré plaquettaire, plasma ; ou encore un médicament dérivé du sang du type notamment protéines coagulantes, immunoglobulines, albumine.

Suivant un mode de réalisation de l'invention, la composition aqueuse comprend des lipides ; ce mode de réalisation est préféré lorsque le lipide utilisé est plus soluble dans la composition aqueuse que dans la phase intermédiaire.

Dans un mode de réalisation, la phase intermédiaire est une dispersion de molécules amphiphiles, de préférence de lipides, dans une composition dont la densité est inférieure à celle de l'eau, qui peut être notamment une huile minérale ou un mélange d'huiles minérales, un alcane ou un mélange d'alcanes, un alcène ou un mélange d'alcènes, un terpène ou un mélange de terpène ou d'autres solvants comme le chloroforme, le toluène ou un alcool (méthanol, éthanol). Avantageusement, la composition est du décane, l'hexadécane, le dodécane, le squalène. Selon un mode de réalisation préféré de l'invention, les lipides sont séchés préalablement à leur dispersion dans l'huile, de manière à être aussi exempts que possible, d'eau. De préférence, la dispersion des molécules amphiphiles dans l'huile s'effectue par sonication.
Suivant un mode de réalisation particulier, la phase intermédiaire est une dispersion de lipides dans une huile dont la densité est inférieure à celle de l'eau, lesdits lipides étant placés dans un solvant miscible dans l'huile puis l'ensemble est mélangé à l'huile, ladite dispersion étant ensuite évaporée pour éliminer le solvant et les éventuelles traces d'eau.

Suivant un mode de réalisation particulier de l'invention, la phase aqueuse est une solution saline ou du sérum physiologique, ou une solution comprenant au moins un sucre. Avantageusement, la phase aqueuse est une solution de glucose. Suivant un mode de réalisation préféré, la phase aqueuse est iso-osmotique avec la composition aqueuse.

Suivant l'invention, la composition aqueuse, qui deviendra le coeur de la capsule, est mise en contact avec la phase intermédiaire par injection de gouttelettes de cette composition, ces gouttes étant de taille contrôlée et homogène, à une distance fixe et déterminée de l'interface.
Suivant un mode de réalisation particulier de l'invention, le moyen d'injection de gouttes de composition aqueuse de taille contrôlée dans la phase intermédiaire est un ou plusieurs capillaires, de 2 à 50 microns. Avantageusement, ces capillaires sont formés à l'aide d'une étireuse de micropipette puis l'extérieur du capillaire est rendu hydrophobe par tout moyen approprié. Suivant un mode de réalisation particulier de l'invention, l'extrémité du capillaire est introduite dans la phase intermédiaire à proximité de l'interface air-phase intermédiaire et la composition aqueuse contenue dans le capillaire est injectée à un taux de 100 à 500 µl/h, de préférence 250µl/h.

Les gouttelettes injectées sont soumises à la force centrifuge due à la rotation de la chambre et, sous l'action de cette force centrifuge, effectuent un trajet dans la chambre, dit « vol ». Suivant un mode de réalisation de l'invention, pendant le vol, les gouttelettes se recouvrent des molécules amphiphiles dispersées dans la phase intermédiaire. Le temps de vol, c'est-à-dire le temps pour la gouttelette de passer dans la phase intermédiaire entre le moment où elle est arrachée du capillaire jusqu'à ce qu'elle entre en contact avec l'interface, est contrôlé par la force centrifuge. Le temps de vol dépend aussi du rayon de la gouttelette, de la viscosité de la phase intermédiaire, et de l'épaisseur de la phase intermédiaire. Le contrôle du temps de vol permet d'obtenir une bonne qualité de capsules, à savoir sphériques et sans défaut de l'enveloppe.

Ainsi, selon l'invention, le procédé est optimisé en fonction de la taille de la chambre, et en fonction du temps de vol nécessaire à une bonne couverture de la gouttelette. Ainsi, la détermination de la vitesse optimisée de rotation de la chambre est déterminée par essais successifs, très simples à mettre en oeuvre pour l'homme du métier.

Les capsules terminent le vol lorsqu'elles entrent en contact avec l'interface puis, elles traversent l'interface : durant cette traversée, dans un premier mode de réalisation, elles se couvrent d'une seconde couche de molécules amphiphiles, pour former des capsules bi-couche amphiphile.

Dans un second mode de réalisation, la capsule entraine de la phase intermédiaire lors de sa traversée de l'interface pour former des capsules épaisses.

Une fois l'interface traversée, les capsules se trouvent dans la phase aqueuse, de laquelle elles sont récupérées, par tout moyen adéquat.

Avantageusement, le procédé selon l'invention est un procédé à haut rendement, c'est-à-dire permettant une fréquence de production de 1 à 1000 Hz, de préférence de 500 à 1000 Hz. Des fréquences plus élevées peuvent être obtenues par simple adaptation du procédé.

La fréquence d'émission des gouttelettes est optimisée en fonction du temps de vol et du temps de passage de l'interface, pour éviter la coalescence, notamment en vol et au niveau de la surface de l'interface.

Dans le mode de réalisation de l'invention utilisant des capillaires, les gouttes sont arrachées des capillaires à intervalles réguliers.

La force centrifuge a pour effet de faire varier l'épaisseur de l'enveloppe ; elle détermine également le temps de vol et le temps de passage à travers l'interface, et enfin a une influence sur le passage à travers l'interface en tant que tel.

L'invention pourra être mieux comprise à la lecture de la description qui va suivre qui illustre non limitativement l'invention et se lit en regard des figures 1, 2 et 3.
La Figure 1 est un schéma de la chambre de fabrication des capsules en vue de dessus et en vue de côté et se lit en référence à l'exemple 1.
La Figure 2 est un schéma de la chambre de fabrication des capsules en vue de dessus et en vue de côté et se lit en référence à l'exemple 2.
La Figure 3 est un graphe montrant la polydispersité des suspensions de capsules susceptibles d'être obtenues par le procédé selon l'invention.

### Exemple 1

### Dissolution des lipides et évaporation des traces d'eau

Les lipides (Egg Phosphatidyl Choline) sont dissous dans 2 ml de méthanol puis évaporé sous une pression de 200mbar pendant 5-10 minutes à une température de 40°C. Une fois que le ballon est recouvert d'un film homogène de lipides, l'évaporation est poursuivie pendant une heure sous la pression de 100mbar (et une température de 40°C). L'huile minérale (SIGMA® M3516) est alors ajoutée à des lipides (Egg Phosphatidyl choline) à une concentration de 0,5 mM. Pour disperser les lipides, la solution est soniquée dans un bain à la température de 40°C.

### Préparation des capillaires

Des capillaires entre 2 et 50 microns sont formés à l'aide d'une étireuse de micropipette puis sont silanisés en trempant la pointe capillaire dans une solution de silane (0,1% de [3-(triméthoxysilyl) propyl] octadecyldimethylammonium chlorure est ajouté à 90% de méthanol, 10% d'eau mélange) pendant 2 minutes en utilisant un flux d'azote par l'intermédiaire du capillaire pour éviter que sa partie intérieure ne se silanise. Cette silanisation a pour fonction de rendre l'extérieur du capillaire hydrophobe.

### Description de la chambre

La chambre dans laquelle sont formées les capsules est composée d'une boîte de Pétri de 4cm de diamètre refermée hermétiquement par collage, et dont la partie supérieure est munie d'un orifice de 1cm afin de permettre l'entrée du capillaire.

### Production des capsules

La chambre est fixée sur un moteur en rotation (ici un moteur permettant de passer de 5 à 70 tours par seconde). La chambre est en rotation à 10 tours par seconde, puis remplie successivement avec 1,5 ml de solution de glucose, 5 ml de solution de lipides, qui forment instantanément une interface verticale en raison de la force centrifuge. Le capillaire est ensuite introduit dans la solution de lipides dans l'huile, à proximité de l'interface air-huile et une solution de sucrose est alors injectée à partir du capillaire à une pression allant de 80 mbar à 500 mbar ou de façon équivalente, à un taux de 250µl/h.

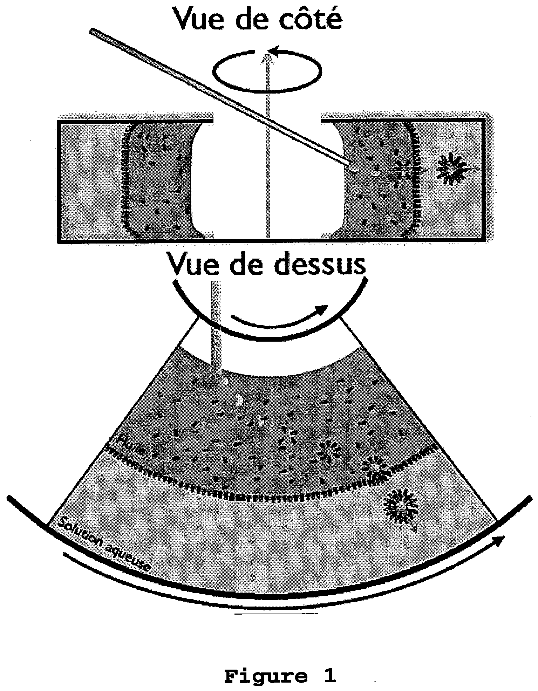

Sur la Figure 1 est représentée une chambre contenant les deux couches de fluides (solution aqueuse, huile). La chambre est en rotation autour de son axe de révolution à une fréquence pouvant aller de 5 à 70 tours par secondes. Les fluides se retrouvent donc sous forme de couches verticales superposées, de la plus dense (la plus excentrée) à la moins dense. La Figure 1 illustre également le principe de formation des capsules selon l'invention: des gouttes sont tout d'abord produites par injection d'une composition aqueuse dans la phase intermédiaire en mouvement de rotation, puis la goutte est poussée par la force centrifuge au travers de l'interface phase intermédiaire-phase aqueuse, pour devenir une capsule.

### Exemple 2

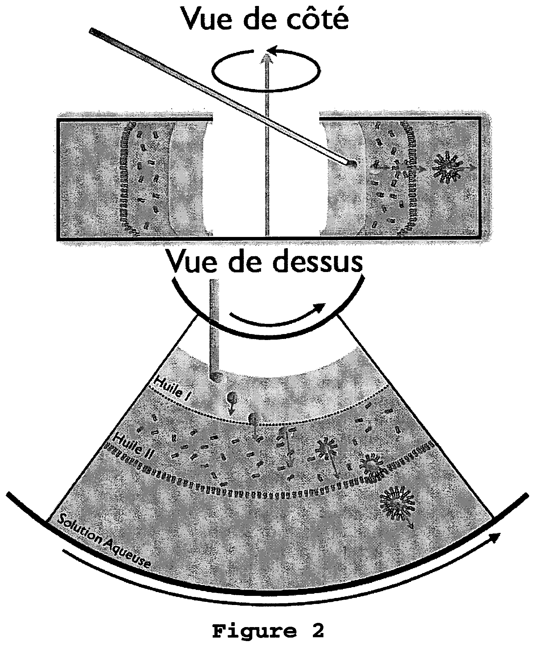

Dans la Figure 2 est représenté un mode de réalisation particulier de l'invention, qui utilise une couche d'huile supplémentaire, moins dense et moins visqueuse que l'autre couche d'huile, permettant d'assurer un goutte-à-goutte dominé par la tension interfaciale (régime capillaire).

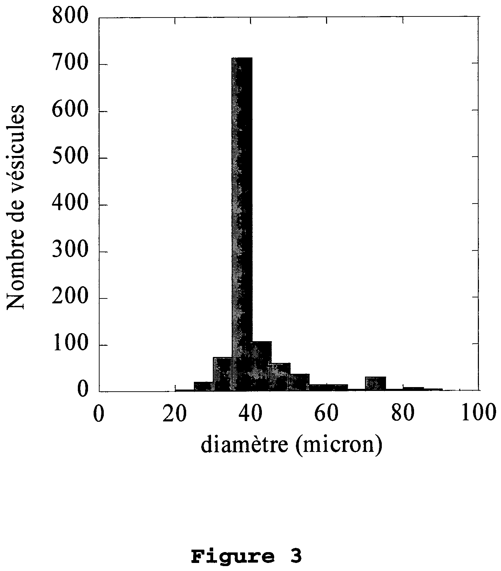

Ainsi, pour une distribution de taille très étroite, il a été utilisé une couche supplémentaire de 1,5 ml d'une autre huile (le décane), de viscosité et de densité inférieure à celle de l'huile minérale et dans laquelle a été injecté la solution de sucrose. Une viscosité plus faible, assure que la production des gouttelettes par goutte à goutte est régie par la tension interfaciale. Par exemple, le nombre capillaire pour des gouttelettes d'eau libérées dans l'huile minérale à 30 tours par seconde est égale à 0,14, alors qu'il n'est que de 0,004 si les gouttes sont injectées dans du décane. Les gouttelettes sont formées de façon séquentielle, recouvertes par les lipides au cours de leur trajet (vol) au travers la couche de solution lipidique, avant de traverser l'interface solution d'huile-glucose, au cours de laquelle elles se recouvrent d'une couche supplémentaire de lipides et deviennent alors des capsules, dans notre cas remplie de sucrose, et dispersées dans du glucose. La Figure 3 indique la distribution de taille de capsules obtenue avec un capillaire de 10 microns, une vitesse de 30 tour/sec (x40 g), et une injection à partir du capillaire dans du décane à 400mBar.

## Revendications

1. Procédé de fabrication en continu et en une seule étape d'une suspension de capsules ayant un diamètre de 1 à 100 microns et un indice de polydispersité inférieur à 10%,
ledit procédé étant mis en oeuvre dans une chambre en rotation contenant une phase aqueuse et une phase intermédiaire,
ledit procédé comprenant la mise en contact d'une composition aqueuse, qui deviendra le coeur de la capsule, avec ladite phase intermédiaire par injection de gouttelettes de cette composition aqueuse, ces gouttelettes étant de taille contrôlée et homogène, à une distance fixe et déterminée de l'interface entre ladite phase intermédiaire et ladite phase aqueuse, au travers de laquelle lesdites gouttelettes sont forcées sous l'effet de la force centrifuge générée par la rotation de la chambre,
puis la récupération d'une suspension aqueuse de capsules.

2. Procédé selon la revendication **1,** dans lequel la phase intermédiaire est une dispersion de molécules amphiphiles dans un solvant non miscible à l'eau et de densité inférieure à celle de l'eau.

3. Procédé selon l'une quelconque des revendications **1** ou **2,** dans lequel la phase aqueuse a une densité inférieure à celle de la composition aqueuse et la phase aqueuse et la composition aqueuse sont iso-osmotiques.

4. Procédé selon l'une quelconque des revendications **1** à **3,** dans lequel la composition aqueuse est injectée au moyen de capillaires à un débit de 100 à 500 µl/h ou à une pression fixe de 80 à 500 mbar dans la phase intermédiaire se trouvant dans la chambre en rotation, à une distance fixe et déterminée de l'interface, ladite injection résultant en la production de gouttelettes à intervalles réguliers dans la phase intermédiaire.

5. Procédé selon l'une quelconque des revendications **1** à **4,** dans lequel la polydispersité des gouttelettes produites est dépendante du nombre capillaire de l'écoulement à l'extrémité du capillaire.

6. Procédé selon l'une quelconque des revendications **2** à **5,** dans lequel les molécules amphiphiles dispersées dans la phase intermédiaire sont choisies parmi les lipides.

## Patentansprüche

1. Verfahren zur kontinuierlichen Herstellung einer Suspension für Kapseln mit einem Durchmesser von 1 bis 100 Mikron und einem Polydispersitätsindex kleiner 10% in einem einzigen Arbeitsschritt,
wobei das Verfahren in einer rotierenden Kammer ausgeführt wird, welche eine wässrige Phase und eine Zwischenphase umfasst,
wobei das Verfahren das In-Kontakt-Bringen einer wässrigen Zusammensetzung, die der Kern der Kapsel wird, mit der Zwischenphase durch Injektion von Tröpfchen dieser wässrigen Zusammensetzung umfasst, wobei die Tröpfchen eine kontrollierte homogene Größe aufweisen, mit einem festen und festgelegten Abstand zur Schnittstelle zwischen der Zwischenphase und der wässrigen Phase, durch welche die Tröpfchen unter Einwirkung einer durch die Drehung der Kammer erzeugten Zentrifugalkraft hindurch gezwungen werden,
sowie dann die Gewinnung einer wässrigen Kapselsuspension.

2. Verfahren nach Anspruch **1,** bei welchem die Zwischenphase eine Dispersion amphiphiler Moleküle in einem Lösungsmittel ist, welches nicht mit Wasser mischbar ist und eine geringere Dichte aufweist als Wasser.

3. Verfahren nach einem der Ansprüche **1** oder **2,** bei welchem die wässrige Phase eine geringere Dichte aufweist als die wässrige Zusammensetzung und wobei die wässrige Phase und die wässrige Zusammensetzung iso-osmotisch sind.

4. Verfahren nach einem der Ansprüche **1** bis **3,** bei welchem die wässrige Zusammensetzung mittels Kapillaren mit einem Durchfluss von 100 bis 500 µl/h oder mit einem festen Druck von 80 bis 500 mbar in die Zwischenphase injiziert wird, die sich in der rotierenden Kammer befindet, mit einem festen und festgelegten Abstand zur Schnittstelle, wobei die Injektion zur Erzeugung von Tröpfchen mit regelmäßigen Abständen in der Zwischenphase führt.

5. Verfahren nach einem der Ansprüche **1** bis **4,** wobei die Polydispersität der erzeugten Tröpfchen von der Kapillarzahl des Stroms am Ende der Kapillare abhängt.

6. Verfahren nach einem der Ansprüche **2** bis **5,** wobei die in der Zwischenphase dispergierten amphiphilen Moleküle aus Lipiden gewählt werden.

## Claims

1. A one-step continuous process for manufacturing capsules having a diameter from 1 to 100 microns and a polydispersity index of less than 10%,
said process being performed in a rotating chamber containing an aqueous phase and intermediate phase,
said process comprising the placing in contact of an aqueous composition, which will become the core of the capsule, with said intermediate phase by injecting droplets of this aqueous composition, these droplets having a controlled and homogeneous size, at a fixed and determined distance from the interface between said intermediate phase and said aqueous phase, through which said droplets are forced under the effect of the centrifugal force generated by the rotation of the chamber,
followed by the recovery of an aqueous suspension of capsules.

2. The process as claimed in claim **1,** in which the intermediate phase is a dispersion of amphiphilic molecules in a water-immiscible solvent with a density less than that of water.

3. The process as claimed in either of claims **1** and **2,** in which the aqueous phase has a density less than that of the aqueous composition and the aqueous phase and the aqueous composition are isoosmotic.

4. The process as claimed in any one of claims **1** to **3,** in which the aqueous composition is injected via capillaries at a flow rate of 100 to 500 µl/h or at a fixed pressure of 80 to 500 mbar into the intermediate phase that is in the rotating chamber, at a fixed and determined distance from the interface, said injection resulting in the production of droplets at regular intervals in the intermediate phase.

5. The process as claimed in any one of claims **1** to **4,** in which the polydispersity of the droplets produced is dependent on the capillary number of the flow at the end of the capillary.

6. The process as claimed in any one of claims **2** to **5,** in which the amphiphilic molecules dispersed in the intermediate phase are chosen among lipids.
